# EUROPEAN PATENT SPECIFICATION

(11) **EP 1 414 458 B1**
(45) Date of publication and mention of the grant of the patent: **11.04.2007**
(21) Application number: 02746895.8
(22) Date of filing: 03.07.2002
(51) Int. Cl.: A61K 31/485, A61K 9/00, A61P 25/04

(54) **ORAL ADMINISTRATION OF 6-HYDROXY-OXYMORPHONE FOR USE AS AN ANALGESIC**
ORALE GABE VON 6-HYDROXY-OXYMORPHON ALS ANALGETIKUM
ADMINISTRATION ORALE DE 6-HYDROXY-OXYMORPHONE UTILISE EN TANT QU'ANALGESIQUE

(30) Priority: 06.07.2001 US 303357 P; 15.10.2001 US 329445 P; 15.10.2001 US 329432 P; 15.10.2001 US 329444 P
(43) Date of publication of application: 06.05.2004
(73) Proprietor: Endo Pharmaceuticals Inc., Chadds Ford, PA 19317 (US)
(72) Inventor: KAO, Huai-Hung, Syosset, NY 11791 (US); SMITH-CARLISS, Richard, Westchester, PA 19382 (US); MCCALL, Troy, Germantown, TN 38138 (US); LEE, David, Chadds Ford PA 19317 (US)
(74) Representative: Buckley, Guy Julian
(86) International application number: PCT/US2002/021400
(87) International publication number: WO 2003/004032

(56) References cited:
- US-A- 4 366 159
- US-A- 4 844 907
- US-A- 5 629 011
- US-A- 6 166 211
- CONE, EDWARD J.: "General procedure for the isolation and identification of 6-.alpha.- and 6-.beta.-hydroxy metabolites of narcotic agonists and antagonists with a hydromorphone structure" J. CHROMATOGR. (1976), 129, 355-61 , XP001106444
- CONE E J ET AL: "Oxymorphone metabolism and urinary excretion in human, rat, guinea pig, rabbit, and dog." DRUG METABOLISM AND DISPOSITION, (1983 SEP-OCT) 11 (5) 446-50. , XP001106446
- PLUMMER ET AL.: "Influence of polarity on dose-response relationships of intrathecal opioids in rats" PAIN, vol. 40, 1990, pages 339-347,

## Description

### Field of Invention

The invention relates to a use of 6-hydroxy oxymorphone for the manufacture of a composition treating pain wherein said pharmaceutical composition is to be delivered by oral administration.

Cone, Edward J; "General procedure for the isolation and identification of 6-alpha and 6-Beta hydroxyl metabolites of narcotic agonists and antagonists with a hydromorphone structure", J. Chromatography, (1976), 129, 355-61 discloses the isolation and identification of certain 6-alpha and 6-Beta hydroxyl metabolites of narcotic agonists and antagonists using thin layer chromatography and gas chromatography. Some of the disclosed compounds are indicated to have narcotic activity.

US-A-5 629 011 discloses a composition for nasal administration of polar metabolites of opioid analgesics comprising a polar metabolite of an opioid analgesic and an absorption promoting agent. Preferred metabolites are disclosed as morphine-6-glucuronide and morphine-6-sulphate.

John L. Plummer, et al.; Pain, 40(1990) 339-347; Elsevier, "Influence of polarity on dose-response relationships of intrathecal opioids in rats"; speculates that, in humans, opioids more polar than morphine, such as 6-hydroxyoxymorphone, may be useful where prolonged pain relief following inrathecal administration is desired.

### Summary of the Invention

The present invention is as set out in the accompanying claims. The present invention provides a use of 6-hydroxy oxymorphone for the manufacture of a pharmaceutical composition for treating pain wherein said pharmaceutical composition is to be delivered by oral administration. To achieve the desired analgesic effect, blood plasma levels of 6-hydroxy oxymorphone have to be raised to about 0.2 ng/mL, most preferably to about 0.3 ng/mL during treatment a use of 6-hydroxy oxymorphone for the manufacture of pharmaceutical compositions comprising one or more carriers, diluents, and excipients for treating pain wherein said pharmaceutical composition is to be delivered by oral administration is also provided.

### Brief Description of the Drawings

Fig. 1 is a pharmacokinetic profile for 6-hydroxy oxymorphone with PID scores.
Ref Fig. 2 is a pharmacokinetic profile for oxymorphone with PID scores.
Fig. 3 is a pharmacokinetic profile for 6-hydroxy oxymorphone with categorical pain scores.
Ref Fig. 4 is a pharmacokinetic profile for oxymorphone with categorical pain scores.

### Detailed Description

The uses described herein provide for the administration of a pharmaceutical composition containing 6-hydroxy oxymorphone as an active ingredient. In a preferred embodiment the preferred composition comprises 6-hydroxy oxymorphone alone (excepting, of course, carriers, diluents, and other excipients). In other preferred embodiments, 6-hydroxy oxymorphone may be combined with other opioids or other pharmaceutical agents. For example, another preferred embodiment provides compositions comprising both 6-hydroxy oxymorphone and its parent, oxymorphone.

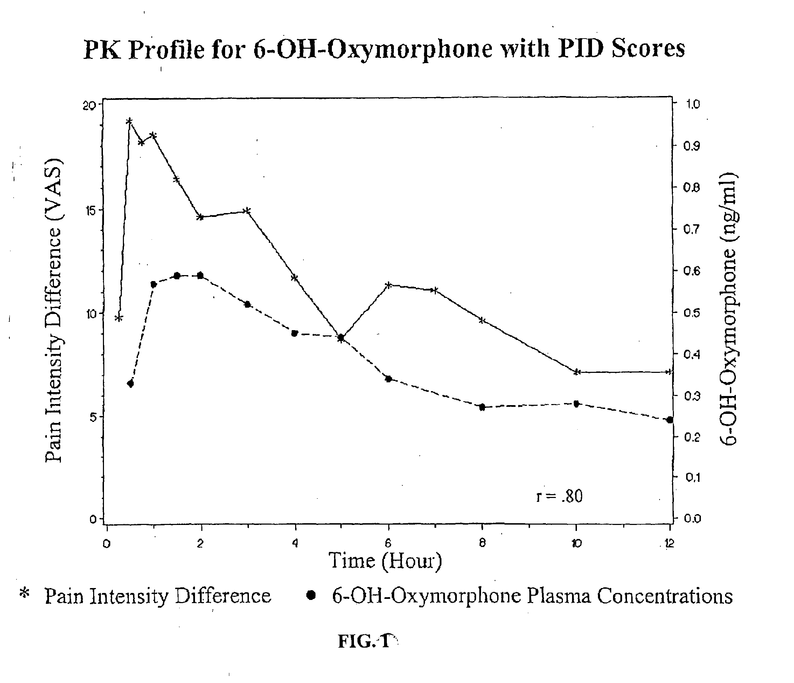

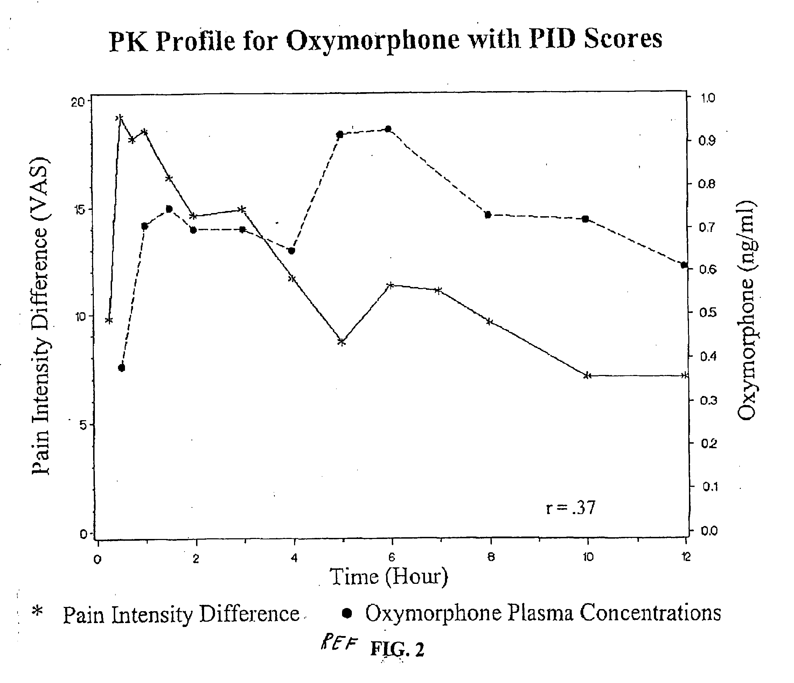

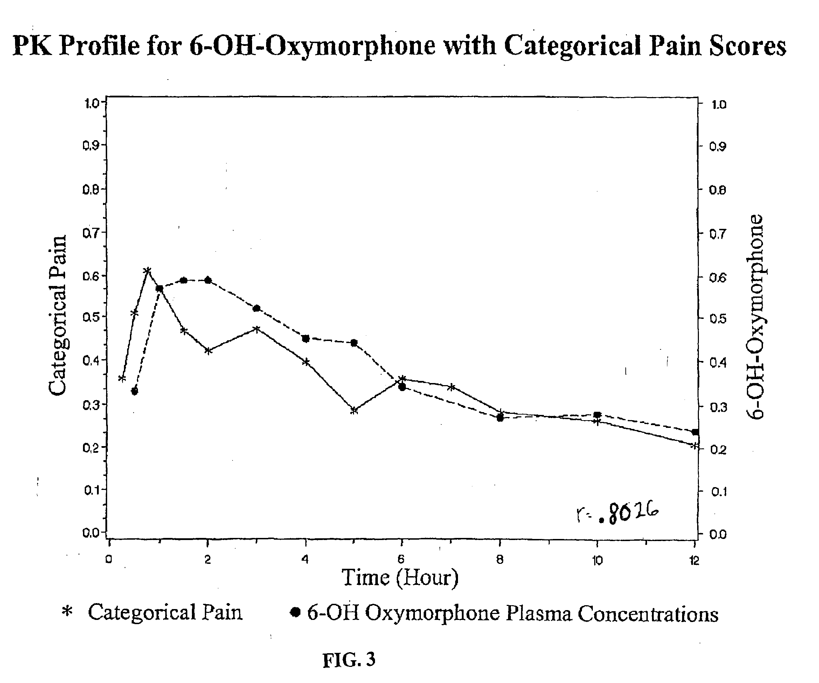

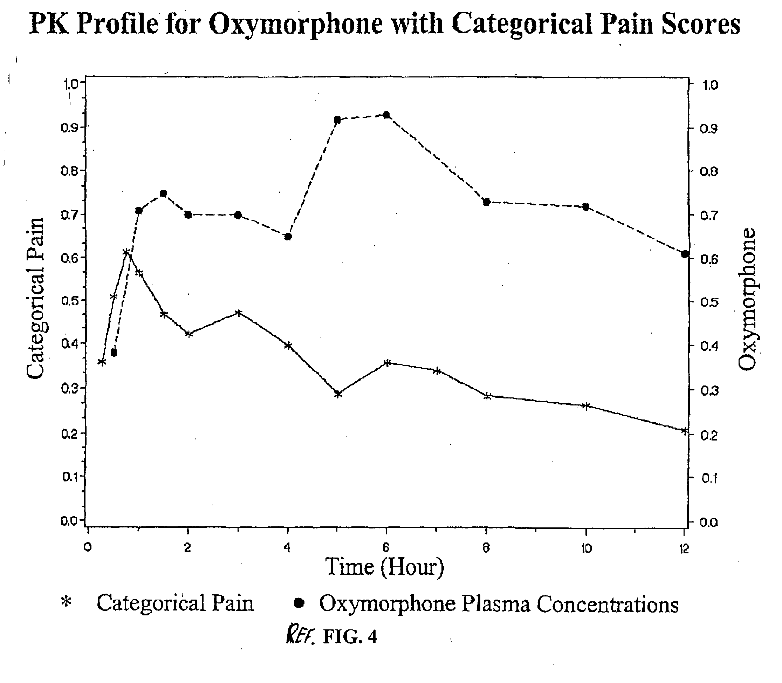

In two separate studies, blood plasma levels and indications of pain relief were recorded over a 12 hour period after. Figs. 1-4 show graphical representation of the data combining the two studies such that the effect of blood plasma levels of oxymorphone and its metabolite, 6-hydroxy oxymorphone on pain can be evaluated.

The administration of oxymorphone yields blood plasma levels of oxymorphone and all of its metabolites, 6-hydroxy oxymorphone. Oxymorphone levels peak within 2 hours, fall slightly, and plateau. Interestingly, the level spikes again at 4-6 hours from administration. After this time, oxymorphone levels again drop and eventually fall to levels near the earlier plateau.

Like oxymorphone, 6-hydroxy oxymorphone blood plasma levels peak within 2 hours after administration. After the initial peak, however, a more or less steady decline in the 6-hydroxy oxymorphones plasma levels is observed.

Comparing these levels to the pain profiles, a correlation between the 6-hydroxy oxymorphone blood plasma levels and pain relief can be seen. The pain levels nearly mirror the 6-hydroxy oxymorphone levels, with substantial rises in relief near the spikes associated with 6-hydroxy oxymorphone blood levels. Thus, pain relief can be achieved through administration of 6-hydroxy oxymorphone alone.

In addition to the pharmacokinetic studies, binding studies have been conducted to compare the binding affinity of 6-hydroxy oxymorphone to that of oxymorphone. The results are reported in TABLE 1. These results clearly indicate that 6-hydroxy oxymorphone has great binding affinity for the δ, κ, and µ receptor cites, comparable to the binding affinity of its parent. The inventors believe that by virtue of this binding affinity, 6-hydroxy oxymorphone has similar analgesic effects to its parent, oxymorphone.

**TABLE 1: ASSAY REPORT**

| | **6-HYDROXY OXYMORPHONE** | | **OXYMORPHONE** | |
|---|---|---|---|---|
| | **10nm 1.0 E-8** | **10µm 1.0 E-5** | **10nm 1.0 E-8** | **10µm 1.0 E-5** |
| **Opiate, Delta** 1 | -4.12% | 90.48% | -18.26% | 89.03% |
| **Opiate, Delta** 2 **(Human Recombinant)** | 7.19% | 55.45% | 7.76% | 72.74% |
| **Opiate, Kappa (Human Recombinant)** | 2.45% | 62.47% | 10.35% | 89.41% |
| **Opiate, Mu (Human Recombinant)** | 63.16% | 99.91% | 85.42% | 100.39% |

Accordingly, uses of the metabolite, 6-hydroxy oxymorphone, directly have been developed. It is believed that the β isomer has greater efficacy in the treatment of pain.

Pharmaceutical compositions containing either 6-α-hydroxy oxymorphone,6- β-hydroxy oxymorphone, or mixtures thereof can be used in the invention.

Formulation as a suspension, syrup, or other liquid, tablet, capsule, liquid-filled gel cap, or other solid or semi-solid means may be used. The composition may alternately be in the form of a time release formulation, including timed, suspended and extended release formulations. Regardless of the formulation, an amount of 6-hydroxy oxymorphone for inducing analgesia will be supplied to the patient. Blood plasma levels of 6-hydroxy oxymorphone must be raised to levels sufficient to induce the desired level of analgesia.

The amount administered will be dependent upon normal criteria such as patient weight, intensity of pain, and other factors. Based on the pharmacokinetic studies, blood plasma levels around at least 0.2 ng/mL will provide some analgesia. The upper plasma level limit will be ultimately established by safety concerns. Over-dosing of any opioid, including 6-hydroxy oxymorphone, may lead to respiratory failure and other undesirable side effects and can even result in death. The blood plasma level of 6-hydroxy oxymorphone has to be raised to at least 0.3 ng/mL. Subsequent doses may be required to maintain these blood levels.

The preferred administration is of 6-hydroxy oxymorphone with appropriate carriers and excipients as will be readily apparent to those skilled in the art. The resulting blood plasma in these preferred administrations will therefore be substantially free of oxymorphone.

## Claims

1. Use of 6-hydroxy oxymorphone for the manufacture of a pharmaceutical composition for treating pain, wherein said pharmaceutical composition is to be delivered by oral administration.

2. Use as claimed in claim 1, wherein said pharmaceutical composition is in an oral delivery form selected from a liquid formulation, syrup, suspension, solid formulation, tablet, capsule, liquid-filled gel cap, and a semi-solid formulation.

3. Use as claimed in any one of the preceding claims, wherein said pharmaceutical composition comprises, in addition to said 6-hydroxy oxymorphone, one or more carriers, diluents, and excipients.

4. Use as claimed in any one of the preceding claims, wherein said pharmaceutical composition comprises, in addition to said 6-hydroxy oxymorphone, another pharmaceutical agent.

5. Use as claimed in claim 4, wherein said other pharmaceutical agent is an opioid.

6. Use as claimed in claim 5, wherein said pharmaceutical composition comprises both 6-hydroxy oxymorphone and oxymorphone.

## Patentansprüche

1. Verwendung von 6-Hydroxyoxymorphon für die Herstellung einer pharmazeutischen Zusammensetzung zur Schmerzbehandlung, wobei die pharmazeutische Zusammensetzung durch orale Verabreichung appliziert werden soll.

2. Verwendung wie in Anspruch 1 beansprucht, wobei es sich bei der pharmazeutischen Zusammensetzung um eine orale Applikationsform ausgewählt aus einer flüssigen Formulierung, Sirup, Suspension, festen Formulierung, Tablette, Kapsel, flüssigkeitsgefüllten Gelkapsel und einer halbfesten Formulierung handelt.

3. Verwendung wie in einem der vorhergehenden Ansprüche beansprucht, wobei die pharmazeutische Zusammensetzung zusätzlich zu dem 6-Hydroxyoxymorphon einen oder mehr Träger, Verdünnungsmittel und Arzneiträger umfasst.

4. Verwendung wie in einem der vorhergehenden Ansprüche beansprucht, wobei die pharmazeutische Zusammensetzung zusätzlich zu dem 6-Hydroxyoxymorphon einen weiteren pharmazeutischen Wirkstoff umfasst.

5. Verwendung wie in Anspruch 4 beansprucht, wobei es sich bei dem anderen pharmazeutischen Wirkstoff um ein Opioid handelt.

6. Verwendung wie in Anspruch 5 beansprucht, wobei die pharmazeutische Zusammensetzung sowohl 6-Hydroxyoxymorphon als auch Oxymorphon umfasst.

## Revendications

1. Utilisation d'oxymorphone 6-hydroxy pour la fabrication d'une composition pharmaceutique analgésique, dans laquelle ladite composition pharmaceutique doit être administrée par voie orale.

2. Utilisation selon la revendication 1, dans laquelle ladite composition pharmaceutique se présente sous forme d'administration par voie orale choisie dans une formulation liquide, un sirop, une suspension, une formulation solide, un comprimé, une gélule, une capsule remplie de liquide et une formulation semi-solide.

3. Utilisation selon l'une quelconque des revendications précédentes, dans laquelle ladite composition pharmaceutique comprend, outre ladite oxymorphone 6-hydroxy, un ou plusieurs vecteurs, diluants et excipients.

4. Utilisation selon l'une quelconque des revendications précédentes, dans laquelle ladite composition pharmaceutique comprend, outre ladite oxymorphone 6-hydroxy, un autre agent pharmaceutique.

5. Utilisation selon la revendication 4, dans laquelle ledit autre agent pharmaceutique est un opioïde.

6. Utilisation selon la revendication 5, dans laquelle ladite composition pharmaceutique comprend à la fois de l'oxymorphone 6-hydroxy et de l'oxymorphone.
